Europäisches Patentamt

European Patent Office

Office européen des brevets

(19)

(11) Publication number: **0 253 654**
**A2**

(12) # EUROPEAN PATENT APPLICATION

(21) Application number: **87306272.3**

(22) Date of filing: **15.07.87**

(51) Int. Cl.⁴: **C 07 H 15/252**

(30) Priority: **16.07.86 IT 2114686**

(43) Date of publication of application:
**20.01.88 Bulletin 88/03**

(84) Designated Contracting States:
**AT BE CH DE ES FR GB GR IT LI LU NL SE**

(71) Applicant: **FARMITALIA CARLO ERBA S.p.A.**
**Via Carlo Imbonati n.24**
**I-20159 Milan (IT)**

(72) Inventor: **Turci, Giampiero**
**Via Paolini, 24**
**I-10139 Torino (IT)**

**Varesio, Carlo**
**Via Sismonda, 7**
**I-10100 Torino (IT)**

(74) Representative: **Woods, Geoffrey Corlett et al**
**J.A. KEMP & CO. 14 South Square Gray's Inn**
**London WC1R 5EU (GB)**

(54) Continuous process for transforming 4'-keto-N-trifluoroacetyldaunorubicin into 4'-epi-N-trifluoroacetyl-daunorubicin, an intermediate for preparing an anti-tumour compound.

(57) 4-'Epi-N-trifluoroacetyldaunorubicin, an intermediate in the preparation of the anti-tumor compound 4'-epidoxorubicin, is prepared by a continuous process in which:

(i) a first solution of 4'-keto-N-trifluoroacetyldaunorubicin in methylene chloride-methanol (about 1:2, vol:vol) at a concentration of from 10 to 18 g/l and a second solution of sodium borohydride in ethanol at a concentration of about 2 g/l are prepared;

(ii) the said first and second solutions are cooled to a temperature of from -20°C to -40°C and are then mixed in a volume ratio of first solution : second solution of from 7:1 to 8:1 while maintaining the temperature at from -25°C to from -45°C;

(iii) to the thus obtained mixture sufficient acetone is added to destroy the sodium borohydride in excess; and

(iv) the reaction mass is then neutralised at a temperature of from -10°C to +5°C and the resultant 4'-epi-N-trifluoroacetyldaunorubicin is isolated.

EP 0 253 654 A2

## Description

"CONTINUOUS PROCESS FOR TRANSFORMING 4'-KETO-N-TRIFLUOROACETYL-DAUNORUBICIN INTO 4'-EPI-N-TRIFLUOROACETYLDAUNORUBICIN, AN INTERMEDIATE FOR PREPARING AN ANTI-TUMOR COMPOUND"

The present invention refers to a continuous process for transforming 4'-keto-N-trifluoroacetyldaunorubicin into 4'-epi-N-tri-fluoroacetyl-daunorubicin, a useful intermediate for preparing on effective anti-tumor compound.

4'-epidoxorubicin is a well-known anti-tumor compound described and claimed in U.S. Patent No.4,058,519.

A improved process for producing 4'-epidoxorubicin is described in Italian Patent Application No. 21523A/84, 21.06.84. According to this process, the desired final compound is obtained from the N-trifluoroacetyl-protected daunorubicin by a series of successive reaction steps.

One of the said reaction steps consists of the discontinuous treatment of 4'-keto-N-trifluoroacetyldaunorubicin with NaBH₄ in methanol, the reaction mixture being then neutralized by aqueous HCl, diluted with methylene chloride and dried over Na₂SO₄ to give 4'-epi-N-trifluoroacetyldaunorubicin, which is then further reacted.

In the described transformation of 4'-keto-N-trifluoroacetyl daunorubicin into 4'-epi-N-trifluoroacetyldaunorubicin, a severe drawback occurs due to the dosage of NaBH₄ being critical in respect of the amount of ketone to be reacted with: when the amount of NaBH₄ is in defect compared to the amount of ketone, a portion of 4'-keto-N-trifluoroacetyldaunorubicin remains unreacted, while, on the other hand, 13-dihydro-N-trifluoroaceyldaunorubicin is produced as impurity when the amount of sodium borohydride is in excess compared to the amount of ketone.

In both cases, the drawbacks are remarkable, owing to either the great difficulty to control exactly the amounts of reagents or the high cost of the ketone reagent and the difficulty to remove the possible above mentioned impurity.

The main object of the present invention is to provide a continuous processs for transforming 4'-keto-N-trifluoroacetyl-daunorubicin into 4'-epi-N-trifluoroacetyldaunorubicin, whereby the ratios of reagents (particularly NaBh₄ and Ketone), can be exactly and readily controlled, while optimizing at the highest degree the reaction conditions and avoiding losses of reagents, i.e. allowing, in every instant of the whole reaction time, the desired theoretical amounts of reagents to be practically employed and the substantially complete transformation of the starting reagent into the dedsired final compound to be obtained.

Accordingly the present invention provides a continuous process for transforming 4'-keto-N-tri-fluoro-acetyldaunorubicin into 4'-epi-N-trifluoroacetyldaunorubicin, characterised in that

(i) a first solution of 4'-keto-N-trifluoroacetyltyldaunorubicin in methylene chloride-methanol (about 1:2, vol:vol) at a concentration of from 10 to 18 g/l and a second solution of sodium borohydride in ethanol at a concentration of about 2 g/l are prepared;

(ii) the said first and second solutions are cooled to a temperature of from -20°C to -40°C and are then mixed in a volume ratio of first solution : second solution of from 7:1 to 8:1 while maintaining the temperature at from -25°C to from -45°C;

(iii) to the thus obtained mixture sufficient acetone is added to destroy the sodium borohydride in excess; and

(iv) the reaction mass is then neutralised at a temperature of from -10°C to +5°C and the resultant 4'-epi-N-trifluoroacetyldaunorubicin is isolated.

Preferably, in step (ii) the said first and second solutions are cooled to a temperature of about -20°C while the mixing is carried out at a temperature of about -30°C or lower. In step (iii) typically at least 5 ml of acetone is added per gram of sodium borohydride in the second solution. The neutralisation of the reaction mass in step (iv) preferably is accomplished by 0.01 N HC1. After the neutralisation, the 4'-epi-N-trifluoroacetyl-daunorubicin can be isolated by washing, concentration and subsequent isolation according to known techniques.

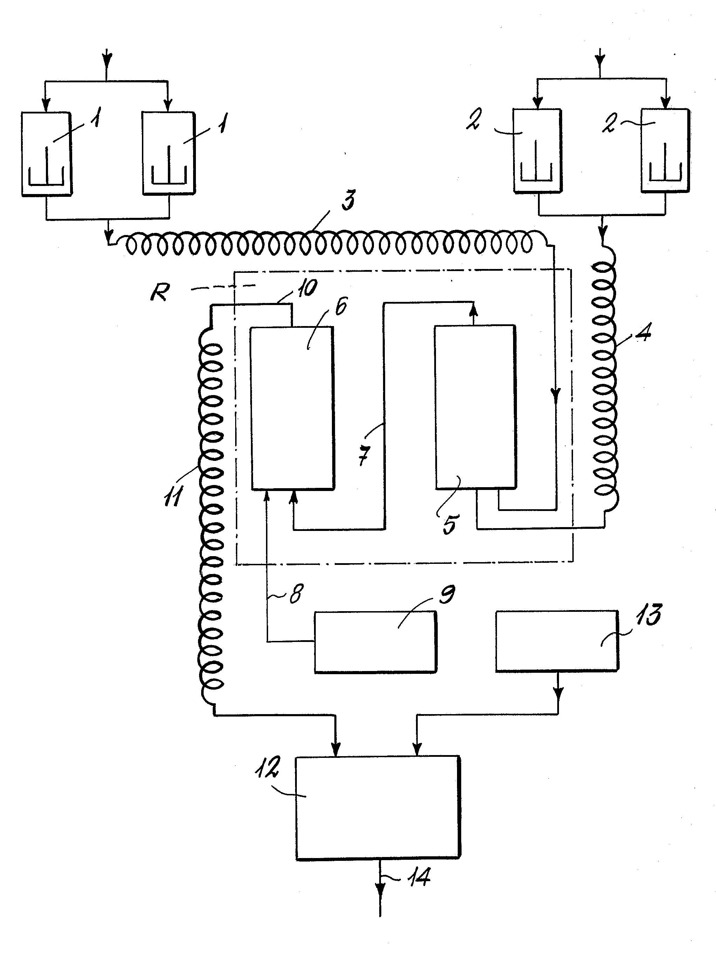

For further explaining the operative procedure and the characteristics of the process according to the present invention, a preferred embodiment thereof will be now described with reference to the accompanying drawing, wherein the only figure shows a block diagram of a plant useful for carrying out the process.

In containers 1, each one provided with stirrers, a first solution of 160 g of 4'-keto-N-trifluoroacetyldaunorubicin (obtained according to the method of Example 1 of Italian Patent Application No. 21523 A/84) in 10 1 of a methylene chloride: methanol mixture (1:2, vol:vol) is prepared.

In other containers 2, also provided with stirrers, a second solution of 2g of NaBH₄ in 1 l of ethanol is prepared.

In the above diagram two separate containers 1 and two separate containers 2 are employed, the first ones being for preparing and cooling the above mentioned solutions to about -20°C to -40°C preferably -30°C, the second ones for feeding the mixing reactor R according to the method which will be described.

First and second solutions from containers 1 and 2 pass through cooling coils 3 and 4, respectively, where the temperature of the solutions is adjusted to between -20°C and -40°C, preferably -30°C.

The two solutions from coils 3 and 4 are fed to the mixing reactor R which preferably, as shown in the Figure, consists of two cylindrical vertical vessels, each having a jacket (not shown, because of known structure) with a flow of cooling fluid therein (e.g. a fluorocarbon such as Fluorinert, trademark, produced and sold by the Italian Firm Montefluos) able to maintain the inner temperature of the two cylindrical vessels 5 and 6 constant below about -30°C, e.g. between -25°C and -45°C.

Preferably, the two cylindrical vertical vessels 5 and 6, acting as mixing reactor, are provided with inner deflecting inclined vanes,such as the types of mixing reactor SMX and SMXL of Sulzer Company.

It can be appreciated that the same cooling fluid can be employed both for maintaining low temperature of the two cylindrical vessels 5 and 6 and for providing the cooling in the coils 3 and 4.

The whole size (particularly the length) of reactor R, i.e. of the two cylindrical vessels 5 and 6, has to be in any case sufficient for securing a perfect and complete mixing of the solutions fed thereto.

As shown in the Figure, the output of coils 3 and 4 is connected to the lower end, or bottom, of cylindrical vessel 5 and the out put of cylindrical vessel 5, at its upper end, is connected to the bottom of cylindrical vessel 6 through a piping 7.

According to an operative, illustrative example, to the bottom of the cylindrical vessel 5 are fed 202 ml/min of the first solution from coil 3 and 27 ml/min of the second solution from coil 4.

To the bottom of cylindrical vessel 6 acetone is fed from a reservoir 9 through piping 8: the amount of acetone fed to cylindrical vessel 6 has to be enough to destroy completely the excess of $NaBH_4$ in the solution flowing in the same cylindrical vessel 6: in practice, the amount of acetone has to be at least 5 ml per 1 gram of $NaBH_4$ dissolved in the second solution.

In the above quantitative example, at least 5,4 ml/min of acetone are to be fed to the cylindrical vessel 6.

A solution of 4'-epi-N-trifluoroacetyldaunorubicin flows out of the top of cylindrical vessel 6, through piping 10 and heat exchanger 11. The heat-exchanger 11 (if any, it can also be placed inside the reservoir 12) acts for heating the solution in order to bring quickly the temperature of the liquid mass in the collection and neutralization reservoir 12 to between -10°C and +5°C.

In reservoir 12 the solution is neutralized to pH 7 (exothermic reaction) by continuously adding O.O1 N HCl from reservoir 13; in this specific example 50 -55 ml/min of O,O1N HCl are added.

HCl is preferred to other acids, because already employed for producing 4'-keto-N-trifluoroacetyldaunorubicin and because it will be subsequently employed (see Italian Patent Application n. 21523 A/84) to transform 4'-epi-N-trifluoroacetyldaunorubicin into 4'-epidoxorubicin.

Out of the neutralization reservoir 12 flow, in continuous through piping 14, 144-152 ml/min of a solution of 4'-epi-N-trifluo-roacetyldaunorubicin. This solution is treated as already described in detail in the Italian Patent Application No. 21523A/84, i.e. washed with deionized water, in discontinuous or, preferably, in continuous in a pulse column or in a column provided with rotating disks, concentrated, still continuously according to usual techniques, and finally the desired 4'-epi-N-trifluoroacetyl-dauno-

rubicin is isolated (e.g. by precipitating it in petroleum ether).

It should be appreciated that the solution prepared in container 1 can contain amounts of the various components different from those indicated above. Thus the concentration of 4'-keto-N-tri-fluoroacetyl-daunorubicin can range from about 10 to about 18 g/l, advantageously from 12 to 16 g/l. In other words the ketone concentration can be remarkably higher than it was possible according to the prior known technique. Also important is to emphasize that sodium borohydride, in container 2, is dissolved in ethanol, wherein the sodium borohydride is more stable than in methanol.

As already mentioned, the temperature in the mixing reactor R must be very low, i.e. at least -25°C or lower.

This allows to obtain two important results: at such a low temperature a good stereospecificity is obtained, the -OH (hydroxy) group of 4'-epi-N-trifluoroacetyldaunorubicin formed in this reactor being in the axial position. In other words, a temperature of -25°C or lower favours the formation of the desired epimer 4'-epi-N-trifluoroacetyldaunorubicin.

The above mentioned favourable conditions do not occur if the reaction is carried out at higher temperature (e.g. -10°C ).

The described reaction, as carried out in reactor R, allows a second important benefit, i.e. the formation of 4'-epi-N-trifluoroacetyldaunorubicin taking place almost instantaneously, while in the moust favourable conditions according to the prior known technique, already mentioned (Italian Patent Application No. 21523 A/84), this same compound requires about ten minutes to be formed.

## Claims

1. A continuous process for transforming 4'-keto-N-trifluoroacetyldaunorubicin into 4'-epi-N-trifluoroacetyldaunorubicin, characterised in that

(i) a first solution of 4'-keto-N-trifluoroacetyldaunorubicin in methylene chloride-methanol (about 1:2, vol:vol) at a concentration of from 10 to 18 g/l and a second solution of sodium borohydride in ethanol at a concentration of about 2 g/l are prepared;

(ii) the said first and second solutions are cooled to a temperature of from -20°C to -40°C and are then mixed in a volume ratio of first solution : second solution of from 7:1 to 8:1 while maintaining the temperature at from -25°C to from -45°C;

(iii) to the thus obtained mixture sufficient acetone is added to destroy the sodium borohydride in excess; and

(iv) the reaction mass is then neutralised at a temperature of from -10°C to +5°C and the resultant 4'-epi-N-trifluoroacetyldaunorubicin is isolated.

2. A process according to claim 1, wherein in step (ii) the temperature is maintained at -30°C or below during the mixing of the said first and second solutions.

3. A process according to claim 1 for 2, wherein in step (iv) the said neutralization of the reaction mass is carried out with 0.01N HCl.

4. A process for the preparation of 4'-epidoxorubicin by converting 4'-epi-N-trifluoro-acetyldaunorubicin thereinto, characterised in that the 4'-epi-N-trifluoroacetyldaunorubicin is prepared by a process as claimed in any one of the preceding claims.

0253654